# EUROPÄISCHE PATENTSCHRIFT

(11) **EP 1 333 782 B1**
(45) Veröffentlichungstag und Bekanntmachung des Hinweises auf die Patenterteilung: **15.09.2004**
(21) Anmeldenummer: 01982034.9
(22) Anmeldetag: 16.11.2001
(51) Int. Cl.: A61F 2/40

(54) **GELENKPROTHESE**
JOINT PROSTHESIS
PROTHESE D'ARTICULATION

(30) Priorität: 16.11.2000 CH 223400
(43) Veröffentlichungstag der Anmeldung: 13.08.2003
(73) Patentinhaber: Horber, Willi, 8005 Zürich (CH)
(72) Erfinder: Horber, Willi, 8005 Zürich (CH)
(74) Vertreter: Walder, Martin Bernhard
(86) Internationale Anmeldenummer: PCT/CH2001/000675
(87) Internationale Veröffentlichungsnummer: WO 2002/039932

(56) Entgegenhaltungen:
- EP-A- 0 024 442
- EP-A- 0 208 578
- EP-A- 0 351 545
- EP-A- 0 663 193
- EP-A- 0 712 617
- EP-A- 0 963 741
- WO-A-99/34756
- DE-C- 19 509 037
- DE-U- 29 918 589
- US-A- 4 318 190

## Beschreibung

### Technisches Gebiet der Erfindung

Die Erfindung betrifft eine Gelenkprothese mit einer über ein Halsteil an einem im Knochen verankerbaren Grundteil kugelgelenkartig angelenkten Kopfkalotte gemäss Oberbegriff des Anspruchs 1.

### Stand der Technik

Aus der EP-A-0 663193 ist eine Hüftgelenkpfanne bekannt, die mit einem Haltering in einer Stützschale an einer am Knochen befestigten Verankerungsplatte gehalten ist Um eine Verdrehung der Hüftgelenkpfanne in der Stützschale zu verhindern, sind in der Stützschale Eindringelemente vorhanden. Wird nun die Hüftgelenkpfanne mit einem Setzinstrument unter Kraftaufwendung in die Stützschale gepresst, so dringen die Eindringelemente in das relativ weiche Kunststoff-Material der Hüftgelenkpfanne ein. Danach wird der Haltering in die richtige Position gebracht, um die Hüftgelenkpfanne in der Stützschale zu halten.

Aus der WO 99/34756 ist eine Gelenkprothese gemäß dem Oberbegriff von Anspruch 1 bekannt, bei welcher in einer halbkugeligen Vertiefung im Schaftteil ein darin kugelgelenkartig verschwenkbares Halsteil angelenkt ist Das Halsteil besitzt eine halbkugelige Anlenkfläche und eine gegenüber einer Achse durch derer Kugelmittelpunkt exzentrischen Konusfläche zum Aufstecken einer Gelenkkalotte. Das Halsteil weist eine von der Kalottenseite her offene Ausbohrung mit einem halbkugeligen Grund. Mit dem Grund wirkt eine in die Ausbohrung eingeführte, durch eine Öffnung im Grund der Ausbohrung hindurch in das Schaftteil einschraubbare Schraube mit Kugelkopf. Die Kugeloberflächen der halbkugeligen Vertiefung im Schaftteil, der Anlenkfläche am Schaftteil, des Grundes der Ausbohrung und des Schraubenkopfes müssen den gleichen Mittelpunkt aufweisen. Die jeweils zwei zusammenwirkenden Hohl- und Vollkugeloberflächen müssen zudem sehr exakt hergestellt und den gleichen Radius aufweisen. Kleinste Abweichungen von den Idealabmessungen führen dazu, dass das Halsteil mit dem Schaftteil nicht genügend fest verbunden werden kann, um eine unbeabsichtigte Verschwenkung des Halsteils gegenüber dem Schaftteil während des Gebrauchs des Gelenks sicher zu verhindern.

Aus der EP-A- 0 712 617 ist eine Humeruskopfprothese bekannt, bei der eine über einen Stiel mit einer Kopfkalotte verbundene Anlenkkugel in einer Grube mit hohlkugeligem Grund an einem Schaftteil angelenkt ist. Zur Fixierung der Anlenkkugel in der Grube sind eine oder mehrere Madenschrauben vorgesehen, die durch das Schaftteil hindurch gegen die Anlenkkugel geschraubt werden können. In einem Ausführungsbeispiel wird mit der Madenschraube die c-förmig aufgeschnittene Anlenkkugel zusammengepresst, um den in einer zentralen Bohrung in der Anlenkkugel steckende Stiel darin festzuklemmen. Es ist in einem anderen Ausführungsbeispiel eine Madenschraube vorgesehen, welche entlang der Stielachse durch die Anlenkkugel und gegen den Grubengrund geschraubt werden kann. Mit dieser Schraube wird die Anlenkkugel gegen die Öffnung der Grube gepresst, welche Öffnung in diesem Ausführungsbeispiel einen gegenüber dem Kugelradius der Grube bzw. der Anlenkkugel kleineren Radius aufweist.

### Aufgabe der Erfindung

Es ist Aufgabe der vorliegenden Erfindung, eine Gelenkprothese vorzuschlagen, bei welcher zum Ausrichten der Position der Kopfkalotte ein gegenüber dem im Knochen verankerbaren Grundteil kugelgelenkartig verschwenkbares Halsteil, auf welches die Kopfkalotte aufsetzbar ist, am Grundteil ausreichend sicher immobilisiert werden kann. Dabei sollen die Herstellungskosten möglichst niedrig und die Anwendung der Prothese durch den Chirurgen möglichst einfach sein.

### Beschreibung der Erfindung

Bei einer Gelenkprothese mit einem im Knochen verankerbaren Grundteil, einem daran angelenkten, eine Halsachse definierenden Halsteil und einem auf der Halsachse liegenden Halsfortsatz am Halsteil und einer daran angeordneten Kopfkalotte ist am Grundteil eine Anlenkvorrichtung ausgebildet, die die Form einer Mulde oder Fläche aufweist An dieser ist ein Anlenkkopf des Halsteils angeordnet Zur Gelenkprothese gehören weiter eine am Halsfortsatz angeordnete Kopfkalotte und wenigstens ein Pressteil zum Anpressen des Anlenkkopfes gegen den Grund der Anlenkvorrichtung. Es sind ferner Mittel zum Verbinden von Pressteil und Grundteil vorgesehen. Grundteil und Kopfkalotte sind zur optimalen Anpassung an die Gegebenheiten des natürlichen Gelenks aus einem Set unterschiedlicher Grundteile bzw. Kopfkalotten ausgewählt Falls die natürliche Gelenkpfanne auch ersetzt wird, weist die Gelenkprothese zusätzlich noch eine künstliche Gelenkpfanne auf. Erfindungsgemäss weist bei einer solchen Gelenkprothese das Pressteil eine Anpressscheibe mit einer Durchtrittöffnung für den Halsfortsatz auf.

Die Anpressscheibe ist gegenüber dem Grundteil um eine Achse durch die Durchtrittöffnung verdrehbar oder unverdrehbar. Die Verdrehbarkeit ist insbesondere dann notwendig, wenn die Anpressscheibe eine Überwurfmutter ist Die Verdrehbarkeit ist auch dann vorteilhaft, wenn die Berührungsbereiche zwischen den Berührungsflächen des Anlenkraumes und des Anlenkkopfes nicht auf einer Kugeloberfläche liegen, sondern z.B. auf einer Zylinderoberfläche oder einer anderen lediglich rotationssymmetrischen Fläche. Bei solcher Anlenkung ist es vorteilhaft, dass sich die Anpressscheibe zusammen mit dem Anlenkkopf um die Achse des zylindrischen Anlenkraumes verdrehbar ist. Dadurch kann eine kugelgelenkartige Verschwenkbarkeit des Halsteils gegenüber dem Grundteil erreicht werden, selbst wenn die Anpressscheibe gegenüber dem axialen Körper des Anlenkkopfes unverdrehbar ist, weil sie beispielsweise mit zwei parallelen Kanten an dessen Zylinderoberfläche anpresst. Die Unverdrehbarkeit hingegen hat den Vorteil, dass beim Anpressen der Anpressscheibe an den Anlenkkopf auf diesen keine Drehkräfte wirken, so dass seine Stellung durch das Anpressen nicht verändert wird.

Ist die Anpressscheibe um eine Achse quer zur Halsachse verschwenkbar am Grundteil betestigt, so muss sie zum Blockieren der Beweglichkeit des Halsteils lediglich einseitig gegen das Grundteil gedrückt werden. Dazu kann z.B. eine einzige Schraube oder ein Spannbügel dienen. Diese Schraube kann somit an einer gut zugänglichen Stelle angebracht sein. Die Achse oder eine andere Anlenkeinrichtung, welche die Verschwenkbarkeit der Anpressscheibe gewährleistet, ist vorteilhaft an einer schlechter zugänglichen Stelle angebracht.

Die Verschraubbarkeit der Anpressscheibe mit dem Grundteil kann durch eine oder mehrere Schrauben erreicht werden, welche durch die Anpressscheibe hindurch in das Grundteil einschraubbar sind. Diese sind von der dem Gelenk zugewandten Seite her zugänglich, welche während des gesamten operativen Eingriffs frei bleibt und erst beim Aufsetzen der Gelenkkalotte auf den Halsfortsatz verdeckt wird. Die Verschraubbarkeit kann auch durch eine Überwurfmutter erreicht werden, welche als Anpressscheibe mit einer Durchtrittöffnung ausgebildet ist, oder welche eine Anpressscheibe gegen das Grundteil presst.

Eine solche Überwurfmutter kann auch einen Bajonettverschluss aufweisen, mit dem sie mit dem Grundteil verbindbar ist Die Anpressscheibe kann auch durch federnde Spannmittel mit dem Grundteil verbindbar oder über eine Klemmverbindung, z.B. ein Konusklemmung, eine Keilklemmung mittels einzuschlagendem Keil oder Exzenterhebel, am Grundteil festklemmbar sein.

Vorteilhaft sind am Anlenkkopf und an der Anpressscheibe vorliegende, zusammenwirkende Berührungsflächen bezüglich Form und Material derart ausgebildet, dass sich ein oder mehrere Berührungsbereiche zwischen diesen Berührungsflächen ergeben, in denen unter Einwirkung der beim Anpressen auftretenden Druckkräfte wenigstens eine der Berührungsflächen plastisch verformbar ist. Die Verformung der Berührungsfläche bewirkt einen Formschluss zwischen Anlenkkopf und Anpressscheibe und somit eine stabile, unverschwenkbare Verbindung der beiden zusammenwirkenden Teile. Die Berührungsbereiche sind dabei vorteilhaft punktuell oder auf einer durchgehenden oder unterbrochenen Linie zwischen Anlenkkopf und Anpressplatte. Die Kleinffächigkeit der Berührungsbereiche begünstigt die plastische Verformung der beteiligten Berührungsflächen. Dasselbe, was für die Berührungsbereiche zwischen Anlenkkopf und Anpressscheibe gilt, gilt auch für die Berührungsbereiche zwischen Anlenkkopf und Anlenkraum.

Vorteilhaft liegen die Berührungsbereiche auf einer Kugeloberfläche, so dass die Anlenkung des Halsteils in jedem Punkt die gesamte Freiheit eines Kugelgelenks gewährt. Zweckmässigerweise ist wenigstens eine der Berührungsflächen durch eine oder mehrere Körperkanten oder eine oder mehrere Körperspitzen gebildet Die andere Berührungsfläche kann durch eine glatte oder rauhe Kugeloberfläche, oder durch Körperkanten oder -spitzen gebildet sein.
Um einer Verdrehung des Halsteils im Anlenkraum entgegenzuwirken, z.B. unter Einwirkung der Drehkraft einer Überwurfmutter auf den Anlenkkopf, sind solche Kanten vorteilhaft quer zu einer Rotationsrichtung des Halsteils um eine Halsachse oder quer zur nach dem Festklemmen grössten auf das Halsteil wirkenden Kraft ausgerichtet.

### Kurzbeschreibung der Figuren

Die Erfindung wird nun anhand von Ausführungsbeispielen erläutert. Es zeigt:
- Fig.1: eine teilweise geschnittene Darstellung einer erfindungsgemässen Schultergelenkprothese mit kugelförmigem Anlenkkopf in einer Anlenkmulde mit einer Kreiskante als Berührungsfläche,
- Fig. 2: eine teilweise geschnittenen Darstellung einer Schultergelenkprothese ohne Kopfkalotte mit gegen einen kugelförmigen Grund der Anlenkmulde gerichteten Spitzen am Anlenkkopf des Halsteils,
- Fig. 3: eine teilweise geschnittene Darstellung einer Schultergelenkprothese mit kugelförmigem Anlenkkopf und sowohl in den Anlenkkopf einpressbaren Kreiskanten als auch einer solchen Spitze im Grund der Anlenkmulde,
- Fig. 4: eine teilweise geschnittene Darstellung des Kopfbereichs einer Schultergelenkprothese mit zylindrischem Anlenkkopf in kugeliger Anlenkmulde,
- Fig. 5: eine teilweise geschnittene Darstellung des Kopfbereichs einer Schultergelenkprothese mit am Grund der Anlenkmulde ausgebildeten Kreiskanten in Berührung mit einer Kugeloberfläche am Anlenkkopf und einer unterbrochenen Kreislinie als Berührungsfläche zwischen auf einem Kreis liegenden Spitzen am Anlenkkopf und einer Kugeloberfläche am Pressteil,
- Fig. 6: eine Schnittdarstellung durch den Kopfbereich einer Schulterprothese mit kugelförmigem Anlenkkopf und kegelförmig ausgebildetem Grund der Anlenkmulde,
- Fig. 7: eine Schnittdarstellung durch den Kopfbereich einer Schulterprothese mit zylindrischem Anlenkkopf mit gerilltem Berührungsbereich in einer zylindrischen Anlenkmulde, gehalten durch eine um die Achse der Anlenkmulde drehbare Anpressscheibe,
- Fig. 8: eine teilweise geschnittene Darstellung des Kopfbereiches einer Schulterprothese mit halbkugeligem Grund der Anlenkmulde und darin einem zu diesem Grund hin zylindrisch geformten Anlenkkopf, welcher zur Anpressscheibe mit einer Kreiskante als Berührungsfläche hin eine Kugeloberfläche aufweist,
- Fig. 9: eine teilweise geschnittene Darstellung einer Schultergelenkprothese mit einem Halsteil gemäss Figuren 10 bis 12 und einem Spannbügel,
- Fig. 10 und 11: . Ansichten von zwei Teilen, die zusammen ein Halsteil bilden,
- Fig.12: eine Aufsicht auf das Halsteil aus den Teilen gemäss Figuren 10 und 11 mit Exzenterring,
- Fig. 13: einen Schnitt durch den Kopfbereich des Schaftteils mit einem Anlenkkopf, welcher in einem Anlenkraum angeordnet ist, der in einem aufschraubbaren Deckel ausgebildet ist.

### Beschreibung der Ausführungsbeispiele

Der Übersichtlichkeit halber werden in der folgenden detaillierten Beschreibung der Ausführungsbeispiele für gleiche und ähnliche Teile die gleichen Bezugsziffern verwendet.

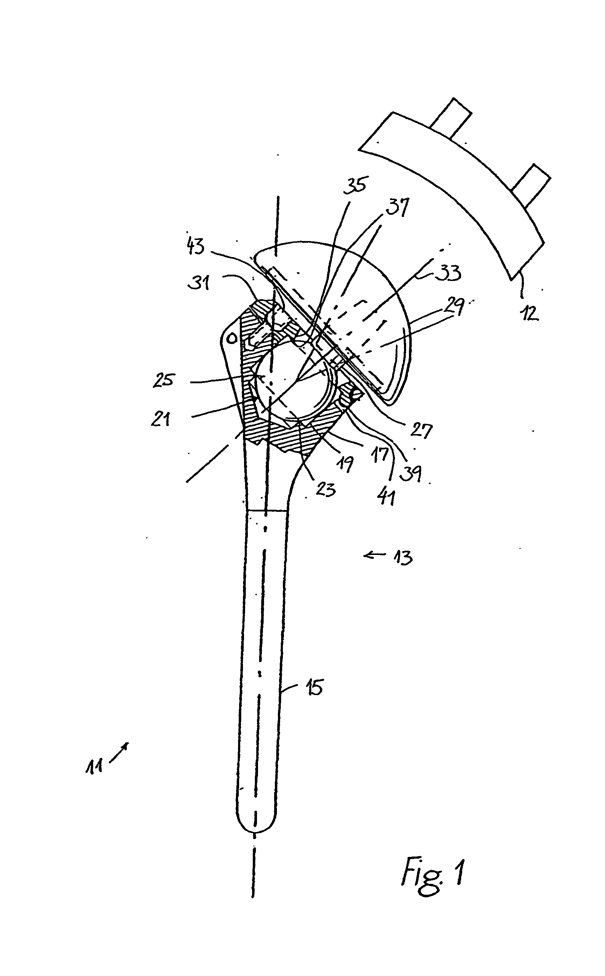

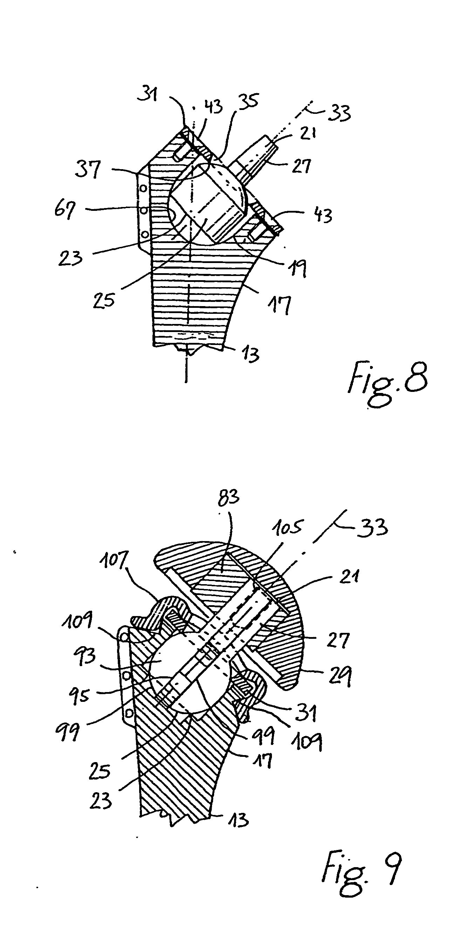

Die in Figur 1 bis 9 dargestellten Schultergelenkprothesen 11 umfassen ein im Humerusknochen eines Patienten verankerbares Grundteil oder Schaftteil 13 mit Schaftstiel 15 und Schaftkopf 17. Im Schaftkopf 17 ist eine Anlenkmulde 19 ausgebildet. Am Schaftteil 13 ist ein Halsteil 21 angelenkt Dieses weist einen in der Anlenkmulde 19 aufliegenden Anlenkkopf 25 und daran einen Halsfortsatz 27 auf. Auf den Halsfortsatz 27 ist eine Kopfkalotte 29 aufgesetzt oder aufsetzbar. Das Halsteil 21 wird durch eine Anpressscheibe 31, oder gemäss Figur 9 mit einer Anpressschraube, gegen den Grund der Anlenkmulde 19 gepresst. In etwa einem Teil der Implantationen von Schultergelenkprothesen wird gleichzeitig die Gelenkpfanne ersetzt In Figur 1 ist das in diesem Fall eingesetzte künstliche Glenoid schematische dargestellt und mit 12 bezeichnet

Eine Halsachse 33 ist durch die Richtung des Halsfortsatzes 27 definiert. In einer Mittelstellung des Halsteils 21 fällt die Halsachse 33 mit der Achse der Anlenkmulde 19 zusammen. Diese Richtung der Halsachse 31 muss bei jedem Patienten individuell eingestellt werden. Um die Halsachse 33 bezüglich Inklination und Ante- bzw. Retroversion, z.B. senkrecht auf die Schnittfläche am Knochen des Patienten, ausrichten zu können, ist das Halsteil 21 in der Anlenkmulde 19 kugelgelenkartig gelagert Die Halsachse ist so um einen Winkel von ca. 20 Grad in jede Richtung aus der Mittelstellung auslenkbar.

In Figur 1 ist ein erstes Ausführungsbeispiel einer Schultergelenkprothese dargestellt, bei welchem eine Kreiskante 23 am Grund der Anlenkmulde 19 ein Auflager für die Kugel des Anlenkkopfes 25 bildet Diese Kreiskante 23 ist gebildet durch die Mündungskante einer zentralen zweiten Bohrung im ebenen Grund einer das Hauptvolumen der Anlenkmulde 19 bildenden ersten Bohrung im Schaftkopf 17. Die grössere erste Bohrung weist einen dem Kugelradius des Anlenkkopfes 21 entsprechenden Radius auf, kann aber auch grösser sein, um das Einsetzen des Anlenkkopfes 25 in die Anlenkmulde 19 zu erleichtern. Die kleinere zweite Bohrung weist einen der Kreiskante 23 entsprechenden Radius auf. Eine Führung der Anlenkkugel 25 wird durch die beiden Kreiskanten 23,37 der kleineren Bohrung und der Durchtrittöffnung 35 erreicht.

In der Anpressscheibe 31 ist eine Durchtrittöffnung 35 für den Halsfortsatz 27 ausgebildet. Ihr Durchmesser kann dem der zweiten Bohrung entsprechen. Die Durchtrittöffnung 35 weist eine kreisrunde Öffnungskante 37 auf, welche am Anlenkkopf 25 anliegt. Auf der einen Seite ist die Anpressscheibe 31 mit einer Scharniernase 39 in einer Schamierausnehmung 41 im Schaftkopf 17 angelenkt. Scharniernase 39 und Schamierausnehmung 41 sind ineinandersteckbar, so dass Anpressscheibe 31 und Schaftteil 13 voneinander lösbar sind. Auf der Schamierseite kann zwischen zwei Scharniernasen 39 eine Schlitzöffnung in der Anpressscheibe 31 ausgebildet sein, so dass die Anpressscheibe 31 hufeisen- oder C-förmig ist Auf der der Scharniernase gegenüberliegenden Seite ist die Anpressscheibe 31 mit einer Schraube 43 mit dem Schaftkopf 17 verschraubt. Dies ergibt eine Dreipunkt-Verspannung zwischen den beiden Scharnieren 39,41 und der Schraube 43.

Liegen die Kreiskanten 23 und 37 an der Kugeloberfläche des Anlenkkopfes 25 an, ohne dass Druck auf diesen ausgeübt wird, so ist das Halsteil 21 kugelgelenkig gegenüber dem Schaftkopf 17 verschwenkbar. Zur Fixierung des Anlenkkopfes 21 im Schaftkopf 17 wird die Schraube 43 angezogen. Damit werden die beiden Kreiskanten 23,37 aufeinander zu bewegt Dabei werden die Kreiskanten 23,37 in die Kugeloberfläche des Anlenkkopfes eingepresst und verformen diesen plastisch. Dadurch wird eine sehr steife Verbindung zwischen dem im Knochen verankerbaren Schaftteil 13 und dem Halsteil 21 erreicht.

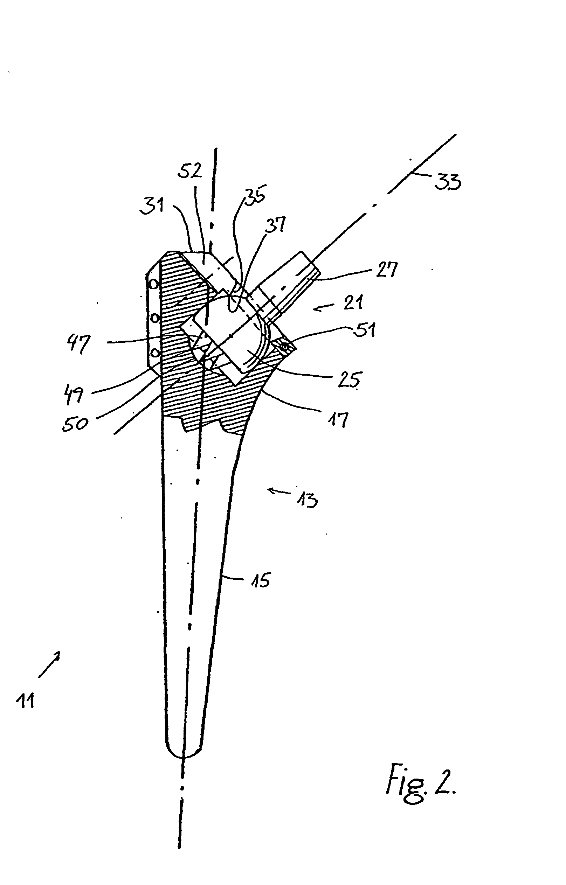

Figur 2 zeigt ein zweites Ausführungsbeispiel einer Schultergelenkprothese 11. Der Anlenkkopf weist einen Kugeloberflächenbereich 45 und drei Spitzkörper 47 auf. Die Spitzen 49 der Spitzkörper 47 weisen denselben Abstand vom Kugelmittelpunkt auf wie die Kugeloberfläche. Die Spitzen 49 sind gegen den Grund der Anlenkmulde 19 gerichtet. Im Schwenkbereich der Spitzen 49 besitzt die Anlenkmulde 19 eine Hohlkugeloberfläche 50 mit demselben Radius wie die Kugeloberfläche des Anlenkkopfes 21. An dieser Hohlkugeloberfläche liegen die Spitzen 49 an. Die Hohlkugeloberfläche 50 ist im Grund einer zylindrischen Anlenkmulde 19 ausgebildet Ein Grosskreis der Kugeloberfläche des Anlenkkopfes 21 liegt an der zylindrischen Mantelfläche der Anlenkmulde 19 an.

Ein Halsfortsatz 27 reicht durch eine Durchtrittöffnung 35 in einer Anpressscheibe 31 hindurch. An ihm wird eine aus einem Set von Kopfkalotten ausgewählte Kopfkalotte befestigt. Die Anpressscheibe 31 ist einseitig mit einer Achse 51 am Schaftkopf 17 angelenkt und gegenüber diesem um die Achse 51 verschwenkbar. Die Anpressscheibe 31 ist C-förmig und weist mit der C-Öffnung 52 von der Achse 51 weg. Dadurch ist es möglich, bei aufgeklappter Anpressscheibe 31 den Anlenkkopf 25 zuerst in die Anlenkmulde 19 einzuführen und hernach die Anpressscheibe zurückzuklappen. Mit je einer Schraube in den beiden C-Schenkeln (es ist lediglich die Achse der Schraube dargestellt) ist die Anpressscheibe 31 mit dem Schaftkopf 17 schraubbar. Die Durchtrittöffnung 35 weist hier wie im ersten Ausführungsbeispiel eine zum in der Anlenkmulde19 liegenden Anlenkkopf 25 gerichtete Öffnungskante 37 auf. Mit dieser Öffnungskante 37 wird beim Anziehen der Schraube 43 der Anlenkkopf 25 gegen den Grund der Anlenkmulde 19 gepresst. Dabei graben sich die Spitzen 49 der Spitzkörper 47 in die Hohlkugeloberfläche 50 der Anlenkmulde 19 ein. Je nach Druckverhältnissen und gewählten Materialien gräbt sich auch die Öffnungskante 37 in die Kugeloberfläche des Anlenkkopfes ein.

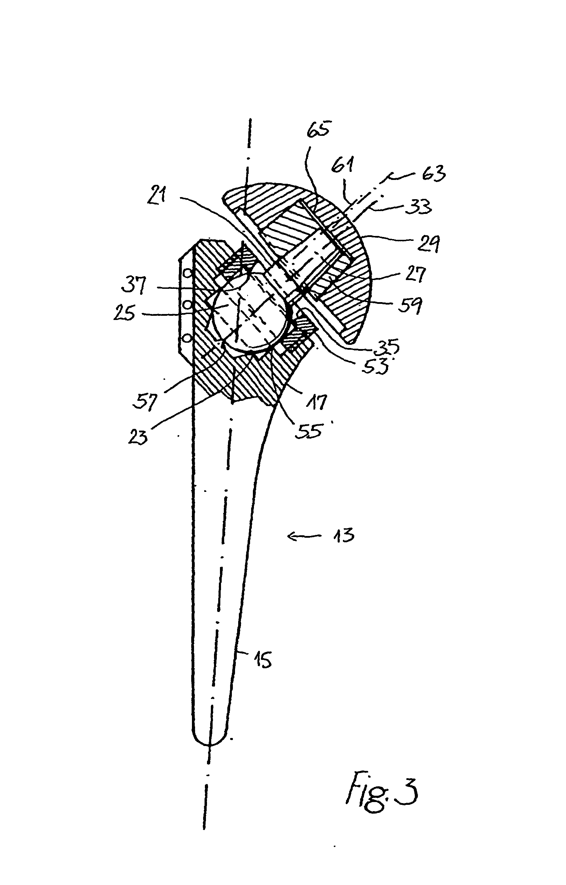

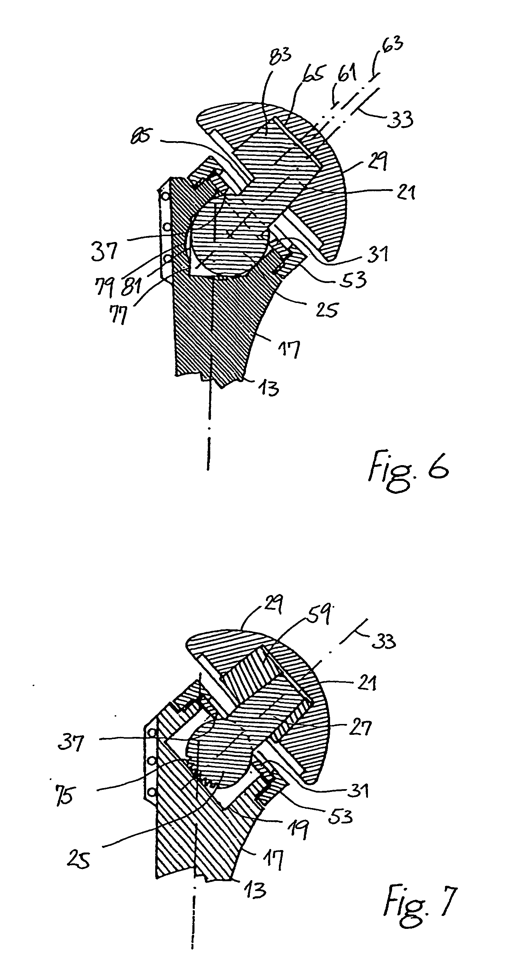

Im in Figur 3 dargestellten dritten Ausführungsbeispiel ist das Pressteil durch eine Überwurfmutter 53 gebildet. Diese ist in ein Gewinde im Rand der Anlenkmulde 19 in die Anlenkmulde 19 einschraubbar. Sie kann auch analog zur Überwurfmutter in den Ausführungsbeispielen gemäss Figur 6 und 7 den Rand der Anlenkmulde 19 umfassen. Hier jedoch liegt die Überwurfmutter auch entlang praktisch eines Grosskreises am kugelförmigen Anlenkkopf 25 an und bildet so eine seitliche Führung für diesen. Die Überwurfmutter greift analog zur Anpressplatte 31 der ersten zwei Ausführungsbeispiele mit dem Öffnungsrand 37 der Durchtrittöffnung 35 für den Halsfortsatz 27 am Anienkkopf 25 an.

Die zylindrisch abgestufte Anlenkmulde 19 weist einen ersten Radius mit dem Gewinde auf. Ein kleinerer zweiter Radius weist beinahe den Radius des Anlenkkopfes 25 auf. Die Mündungskante dieses zweiten Hohlzylinders mit dem zweiten Radius bildet eine erste Kreiskante 55. Der Anlenkkopf wird beim Einpressen in diesen zweiten Hohlzylinder eingepresst, wobei sich zwischen dem Anlenkkopf 25 und der ersten Kreiskante 55 ein Klemmsitz einstellt. Eine zweite Kreiskante 23 mit kleinerem Radius als die erste und eine Körperspitze 57 auf der Achse 33 der Zylinderbohrung am Grund der Anlenkmulde 19 liegen annähernd auf einer Kugeloberfläche mit demselben Radius, wie die Kugel des Anlenkkopfs 25. Beim Einpressen des Anlenkkopfes 25 in die Anlenkmulde 19 kerben diese sich in die Oberfläche des Anlenkkopfes ein.

In Figur 3 ist zudem ein Doppelexzenter zur präzisen Ausrichtung der Kopfkalotte 29 auf die Umrisslinie der Schnittfläche am Knochen dargestellt. Der Doppelexzenter setzt sich zusammen aus einem auf den Halsfortsatz 27 aufsteckbaren Exzenterring 59 mit der Achse 61 und einer exzentrisch zur Kalottenachse 63 in der Kopfkalotte 29 angeordneten Ausnehmung 65 zur Aufnahme des Exzenterringes 59. Zwischen Halsfortsatz 29 und Exzenterring 59, bzw. zwischen letzterem und der Ausnehmung 65 ist ein Klemmsitz vorgesehen. Die Trennung von Halsteil 21 und Exzenterring 59 ist deshalb notwendig, weil der Exzenterring 59 nicht durch die Durchtrittöffnung 37 hindurch passt. Wenn der Halsfortsatz 27 in den Anlenkkopf 25 einsetzbar ist, kann der Exzenterring 59 auch einstückig mit dem Halsfortsatz 27 hergestellt sein.

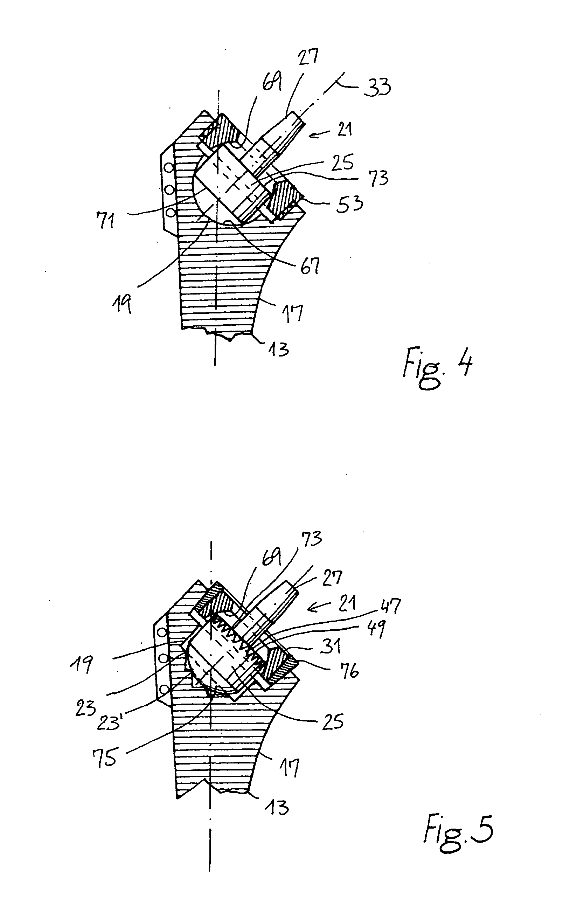

Da die Erfindung unabhängig von der Form des Grundteils oder Schaftteils 13 ist, ist in den Figuren 4 bis 9 lediglich der Schaftkopf 17 mit Halsteil 21 und Pressteil, z.T. mit, z.T. ohne Exzenter und Kopfkalotte 29 dargestellt

Figur 4 zeigt ein viertes Ausführungsbeispiel mit einer Anlenkmulde 19 im Schaftkopf 17 mit etwa halbkugeliger Berührungsfläche 67, einer Überwurfmutter 53 mit konkaver, etwa halbkugeliger Berührungsfläche 69 und einem Halsteil 21 mit zylindrischem Anlenkkopf 25. Der zylindrische Anlenkkopf 25 weist zwei Kreiskanten 71,73 auf, von denen eine mit der Berührungsfläche 67 der Anlenkmulde 19 und die andere mit der Berührungsfläche 69 der Überwurfmutter 53 zusammenwirkt. Die Kreiskanten 71,73 liegen auf einer virtuellen Kugeloberfläche mit etwa dem gleichen Radius wie die Kugeloberflächen 67,69 von Anlenkmulde 19 und Überwurfschraube 53. Zur Justierung des Kugelradius der virtuellen Kugeloberfläche genügt es, die Länge des Anlenkkopfes 25 zu verändern. Der Radius der virtuellen Kugel ist vorteilhaft etwas grösser als der Radius der Hohlkugeloberflächen der beiden Berührungsflächen 67,69. Eine genügende Genauigkeit der Übereinstimmung der beiden Kugelradien ist einfach zu erreichen, da die Berührungsflächen 67,69 bzw. die Kreiskanten 71,73 plastisch verformbar sind.

In Figur 5 ist ein fünftes Ausführungsbeispiel dargestellt. Bei diesem ist die Anpressplatte 31 keine Überwurfmutter mit einem Gewinde, sondern hält mit einem Klemmsitz im Schaftkopf 17. Sie weist eine hohlkugelige Berührungsfläche 69 auf, welche mit einer unterbrochen Kreiskante 73 an einem teilweise zylindrischen Anlenkkopf 25 zusammenwirkt. Die Kreiskante 73 ist durch Einschnitte in den Anlenkkopf 25 zu einer Reihe von Körperspitzen 47 aufgelöst, deren Spitzen 49 sich beim Festschlagen der Anlenkscheibe 31 in den Schaftkopf 17 in die Berührungsfläche 69 eingraben. Die Anlenkmulde 19 weist zwei Kreiskanten 23, 23' auf, welche mit einer Kugeloberfläche 75 am Anlenkkopf 25 zusammenwirken. Auch in diesem Ausführungsbeispiel ist die Übereinstimmung der Radien der Kugeloberfläche 75 und der Kreiskante 73 am Anlenkkopf 25, mit den Radien von virtueller Kugeloberfläche, auf der die Kreiskanten 23,23' liegen und der Berührungsfläche 69 an der Anpressscheibe 31 relativ leicht zu erreichen, da die gewünschte Verformung der Berührungsbereiche grössere Toleranzen zulässt, als es eine Verpressung zweier kongruenter Berührungsflächen kann. Die Verklemmung zwischen Grundteil 13 und Anpressscheibe 31 kann direkt über einen Konus erreicht werden. Besser wird jedoch die Verklemmung der Anpressscheibe 31 wie dargestellt über eine Keilklemmung mit einem z.B. C-förmig die Anpressscheibe umgreifenden Keil 76 erreicht.

Das in Figur 6 dargestellte sechste Ausführungsbeispiel weist einen kegelförmigen Grund 77 der Anlenkmulde 19 und einen kugelförmigen Anlenkkopf 25 des Halsteils 21 auf. Die Mantelfläche des Grundes 77 weist Einschnitte 79 in Richtung der Mantellinien durch die Kegelspitze auf. Dadurch ergeben sich zwischen den Einschnitten 79 Kanten 81 quer zu einer Rotationsrichtung um die Achse 33. Die Anpressscheibe 31 ist mit einer Überwurfmutter 53 am Schaftkopf 17 befestigt. Das Halsteil 21 ist einstückig mit einem Exzenter 83 ausgerüstet. Die Anpressscheibe 31 ist C-förmig. Die C-Öffnung weist eine Weite auf, welche es erlaubt, einen Halsbereich 85 zwischen Anlenkkopf 25 und Exzenter 83 des Halsteils 21 durch diese C-Öffnung hindurch in die Durchtrittöffnung 37 der Anpressscheibe 31 einzuführen. Die Überwurfmutter 53 ist entweder auch C-förmig oder weist einer Öffnungsweite auf, die es erlaubt, die Überwurfmutter 53 über den Exzenter 83 hinab oder über den Anlenkkopf 25 hinauf auf das Halsteil 21 zu schieben. Im letzteren Fall muss zuerst die Überwurfmutter über den Anlenkkopf 25 geschoben und danach die Anpressscheibe 31 zwischen Anlenkkopf 25 und Überwurfmutter 53 eingeschoben werden. Die Anlenkscheibe 31 ist in diesem Beispiel gegenüber der Überwurfmutter 53 verdrehbar. Dies hat den Vorteil, dass beim Anziehen der Überwurfmutter 53 kaum ein Drehmoment auf das Halsteil 21 übertragen wird. Die Öffnungskante 37 wird beim Anziehen der Überwurfmutter 53 gegenüber dem Anlenkkopf 25 nicht verdreht, sondern lediglich gegen diesen gepresst. In nicht festgezogenem Zustand kann das Halsteil 21 um die Halsachse 33 verdreht werden. Dadurch kann der Exzenter 83 in die gewünschte Lage gebracht werden.

In Figur 7 ist ein siebtes Ausführungsbeispiel dargestellt, dessen Anlenkkopf 25 eine zylindrische Form hat Die Anlenkmulde 19 ist ebenfalls zylindrisch. Die Zylinderachse des Anlenkkopfes 25 verläuft senkrecht zur Halsachse 33, während die Zylinderachse der Anlenkmulde 19 senkrecht zur Zylinderachse des Anlenkkopfes 19 steht Der Grund der Anlenkmulde 19 ist eben und senkrecht zu ihrer Zylinderachse. Der Radius der Anlenkmulde erlaubt, den Anlenkkopf darin um iher Zylinderaehse zu verdrehen. Diese Drehung macht die Anpressscheibe 31 jeweils mit Sie liegt mit einer geraden Öffnungskante 37 an der Zylinderoberfläche an und ist kreisrund. Die Anpressscheibe 31 ist durch eine Überwurfmutter 53 am Schaftkopf 17 befestigt und ist zwischen diesen frei verdrehbar. Dadurch kann die stufenlos einstellbare Abweichung der Halsachse 33 von der Zylinderachse der Anlenkmulde 19 stufenlos um 360 Grad gedreht und senkrecht auf die Ebene der Knochenschnittfläche ausgerichtet werden. Dies gibt dieselbe kugelgelenkartige Freiheit der Ausrichtung der Halsachse wie ein echtes Kugelgelenk. Die dem Grund der Anlenkmulde 19 zugerichtete Berührungsfläche 75 des Anlenkkopfes 25 ist gerillt, so dass Kanten auf der Zylinderoberfläche ausgebildet sind, die beim Anziehen der Überwurfmutter 53 im ebenen Grund der Anlenkmulde 19 Furchen einpressen. Durch diese Verkrallung von Anlenkmulde und Anlenkkopf ist sowohl die Verdrehung um die Zylinderachse der Anlenkmulde 19 als auch um die Zylinderachse des Anlenkkopfes 25 blockiert. Bei diesem Ausführungsbeispiel kann die Anpressscheibe auch von der Seite her zwischen die Überwurfmutter 53 und den Anlenkkopf einschiebbar sein. Dazu benötigt die Überwurfmutter kein Gewinde, sondern kann z.B. in eine um die Anlenkmulde umlaufenden Nut eingreifend ausgebildet sein. Bei einer Keilform der Anpressscheibe und einer entsprechend winkligen Berührungsfläche an der Überwurfmutter kann die Pressung auf den Anlenkkopf durch einschlagen der Anlenkscheibe erreicht werden.

Figur 8 zeigt anhand eines achten Ausführungsbeispiels eine sehr einfache Variante der Anpressplatte und eine weitere Möglichkeit der Kombination von zylindrischen und kugelförmigen Körpern und Öffnungen zur Erreichung einer bereits mehrfach ausgeführten kugelgelenkartigen Verbindung zwischen Anlenkmulde und Anlenkkopf bzw. zwischen Schaftkopf 17 und Halsteil 21. In diesem Beispiel weist die Anlenkmulde einen halbkugelförmigen Grund 67 auf, der mit einer Kreiskante 23 eines zylindrischen Anlenkkopfes 25 zusammenwirkt. Zur Anpressscheibe 31 hin ist der Anlenkkopf jedoch kugelig geformt, so dass die kreisrunde Öffnungskante 37 der Anpressscheibe 31 am Anlenkkopf in jeder Schwenkstellung desselben gleichermassen anliegt Die Anlenkscheibe 31 ist mit zwei Schrauben 43 an den Schaftkopf 17 anschraubbar.

Die Ausführungsbeispiele sind ferner dahingehend abwandelbar, dass die Anlenkmulde im Halsteil und der Anlenkkopf im Schaftkopf ausgebildet ist. Der Halsfortsatz muss mit dem Anlenkkopf nicht einstückig ausgebildet sein, sondern kann als ein im Anlenkkopf festmachbarer Stiel vorgesehen sein. Die Ausbildung des Pressteils ist weitgehend unabhängig von der Ausbildung der Berührungsflächen zwischen Anlenkmulde und Anlenkkopf, so dass die unterschiedlichsten Kombinationen unter den dargestellten und erwähnten Ausführungsvariationen möglich sind.

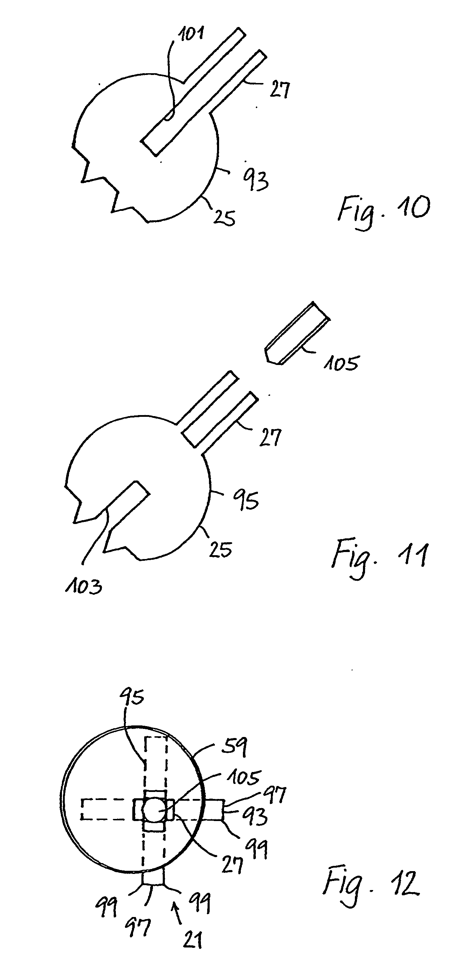

In Figur 9 ist ein neuntes Ausführungsbeispiel dargestellt, in welchem das Halsteil 21 aus zwei Teilen 93,95 zusammengesetzt ist. Das Halsteil 21 entspricht bis auf die Form des Anlenkkopfes 25 exakt dem in Figuren 10 bis 12 dargestellten Halsteil 21. Der Schaftkopf 17 ist mit einer zylindrischen Anlenkmulde 19 ausgestattet, in welcher eine Körperkante 23 ausgebildet ist, an der die Kanten 99 des Halsteils 21 anliegen. Das Halsteil 21 ist mit einer Anpressscheibe 31 gegen den Grund der Anlenkmulde 19 gepresst Die Kraft, mit der die Anpressscheibe 31 gegen den Schaft 13 gepresst wird, ist durch einen Spannbügel 107 auf diese übertragen. Der Spannbügel 107 liegt auf der Aussenseite der Anpressscheibe 31 an und hintergreift einen Hinterschnitt 109 im Schaftkopf 17. Er ist aus einem federnden Material gefertigt, so dass eine relativ grosse Kraft auf die Anpressplatte ausgeübt wird. Der Spannbügel 107 kann auch unmittelbar die Anpressscheibe 31 bilden.

Wie erwähnt muss der Anlenkkopf 25 kein Vollkörper sein. Er kann beispielsweise wie in Figur 10 bis 12 dargestellt, aus Einzelteilen zusammengesetzt sein. Figur 10 und 11 zeigen, die Ansichten von zwei zusammensteckbaren plattenförmigen Teilen 93 und 95. Zusammengesetzt ergeben die Teile 93,95 ein Halsteil 21, das anstelle des Halsteils 21 in Figur 2 eingesetzt werden kann. Die Teile 93,95 sind einfach in der Herstellung. Sie können z.B. mit Laser aus einer Platte geschnitten oder auch gegossen werden. Die Randfläche 97 muss nicht eine Kugeloberfläche sein. Durch die Einpressung in die Anlenkmulde werden die Kanten 99 derart verformt, dass sowohl eine grossflächige Auflage als auch eine sehr gute Verzahnung zwischen Schaftkopf 17 und Halsteil 21 erreicht ist. Der Teil 93 in Figur 10 ist mit dem Schlitz 101 über den Schlitz 103 am Teil 95 in Figur 11 hinweg auf dieses Teil 95 aufschiebbar, so dass alle Kanten 99 auf einer gemeinsamen Kugeloberfläche liegen. Im Halsfortsatz 27 ist in beiden Teilen 93,95 eine Nut ausgebildet, in welche eine Schraube 105 einschraubbar ist.

Der Halsfortsatz 27 aus beiden Teilen 93,93 bildet ein Kreuz mit einer zentralen, quadratischen Ausnehmung. Auf dieses Kreuz ist nun der Exzenterring 59 aufsetzbar. Dank der Kreuzform des Halsfortsatzes 27 und einer entsprechenden Ausnehmung im Exzenterring ist eine Verdrehung des Exzenterringes 59 gegenüber dem Halsteil 21 verunmöglicht. Zur Fixation des Exzenterringes 59 auf dem Halsfortsatz 27 und der beiden Teile 93,95 aneinander kann nun die Schraube 105 eingeschraubt werden, was eine Verklemmung zwischen den Teilen 93,95 einerseits und dem Exzenterring 59 andererseits bewirkt In Figur 12 ist das zusammengesetzte Halsteil 21 mit Exzenterring 59 in einer Aufsicht dargestellt. In dieser Ansicht ist erkennbar, wie die Randflächen 97 der beiden Teile orthogonal zur Plattenebene ausgebildet sind.

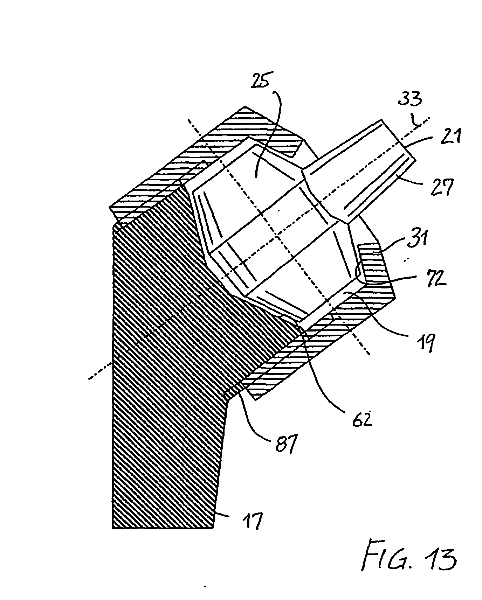

In Figur 13 ist ein Ausführungsbeispiel dargestellt, bei dem der Anlenkraum 19 in einem Deckel ausgebildet ist, welcher Deckel als Überwurfmutter 53 ausgebildet und auf ein am Schaftkopf 17 ausgebildetes Aussengewinde 87 aufgeschraubt ist. Der Anlenkkörper 25 besitzt zwei entgegengesetzt gerichtete Kegelstumpfflächen als Anlenkflächen. Der Boden 62 des Anlenkraumes 19 ist ebenfalls kegelstumpfförmig konkav ausgebildet. Die beiden Winkel der zusammenwirkenden Kegelmantelflächen entsprechen einander komplementär. Die Klemmplatte 31 am Deckel 53 besitzt ebenfalls eine kegelstumpfförmige Klemmfläche 72.

Die Klemmplatte 31 kann auch als Unterlagsscheibe ausgebildet sein, die mit einer Überwurfmutter 53 gegen den Anlenkkörper 25 gepresst wird. Sie muss daher nicht einstückig mit der Überwurfmutter ausgebildet sein. Dies hat den Vorteil, dass beim Anziehen der Überwurfmutter 53 eine Verdrehung der Überwurfmutter sich weniger auf den Anlenkkörper 25 überträgt. Die Klemmflächen des Bodens 62 und/oder der Klemmplatte 31 können auch als eine oder mehrere komplementär kegelstumpfförmig angeordnete ringförmige Kanten ausgebildet sein.

Bei einer Gelenkprothese 11 ist, zusammenfassend gesagt, die Kopfkalotte 29 über ein Halsteil 21 mit einem im Knochen verankerbaren Grundteil 13 verbunden. Das Halsteil 21 ist kugelgelenkartig am Grundteil 13 angelenkt. Dazu ist ein Anlenkkopf 25 am Halsteil 21 in einem Anlenkraum 19 am Grundteil 13 angeordnet und mittels einer Anpressscheibe 31 gegen den Grund des Anlenkraumes 19 gepresst. Diese Anpressscheibe 31 weist eine Durchtrittöffnung 35 für das Halsteil 21 auf.

## Patentansprüche

1. Gelenkprothese (11), mit einer in einem ersten Knochen verankerbaren künstlichen Gelenkpfanne (12) oder zum gelenkigen Zusammenwirken mit einer natürlichen Gelenkpfanne im ersten Knochen, einem axialen, in einem zweiten Knochen verankerbaren Grundteil (13), einem daran angelenkten Anlenkkopf (25), der mit einem daran angeordneten, eine Halsachse (33) definierenden Halsfortsatz (27) zusammen ein Halsteil (21) bildet, welches Halsteil (21) um wenigstens zwei durch den Anlenkkopf (25) gehende, senkrecht zueinander stehende Achsen bezüglich des Grundteils (13) verdrehbar und verschwenkbar ist, einer am Halsfortsatz (27) angeordneten, mit der Gelenkpfanne (12) gelenkig zusammenwirkenden Kopfkalotte (29), wenigstens einem Pressteil (31,53) zum Anpressen des Anlenkkopfes (25) gegen das Grundteil (13), und Mitteln (z.B. 43,53,39,41,51,107) zum Verbinden von Pressteil (31,53) und Grundteil (13), so dass der Halsfortsatz (25) in einer beliebig wählbaren Rotationsstellung und Winkelstellung zur Achse des Grundteils (13) zwischen Pressteil (31,53) und Grundteil (13) feststellbar ist, **dadurch gekennzeichnet,**
**dass** das Pressteil (31,53) eine Anpressscheibe (31,53) mit einer Durchtrittöffnung (37) für den Halsfortsatz (27) aufweist.

2. Gelenkprothese nach Anspruch 1, **dadurch gekennzeichnet, dass** die Anpressscheibe (31) gegenüber dem Grundteil (13) um eine Achse durch die Durchtrittöffnung (35) verdrehbar ist

3. Gelenkprothese nach Anspruch 1, **dadurch gekennzeichnet, dass** die Anpressscheibe (31,53) gegenüber dem Grundteil (13) bezüglich einer Achse durch die Durchtrittöffnung (35) unverdrehbar ist

4. Gelenkprothese nach einem der Ansprüche 1 bis 3, **dadurch gekennzeichnet, dass** die Anpressscheibe (31) um eine Achse quer zur Halsachse (33) verschwenkbar am Grundteil (13) befestigt ist

5. Gelenkprothese nach einem der Ansprüche 1 bis 4, **dadurch gekennzeichnet, dass** Anpressscheibe (31,53) und Grundteil (13) miteinander verschraubbar sind.

6. Gelenkprothese nach Anspruch 5, **dadurch gekennzeichnet, dass** die Anpressscheibe eine Überwurfmutter (53) ist oder mit einer Überwurfmutter (53) befestigbar ist

7. Gelenkprothese nach Anspruch 6, **dadurch gekennzeichnet, dass** die Überwurfmutter (53) mit einem Bajonettverschluss mit dem Grundteil (13) verbindbar ist

8. Gelenkprothese nach einem der Ansprüche 1 bis 5, **dadurch gekennzeichnet, dass** die Anpressscheibe (31) durch federnde Spannmittel (107) mit dem Grundteil (13) verbindbar ist

9. Gelenkprothese nach einem der Ansprüche 1 bis 4, allenfalls in Verbindung mit Anspruch 8, **dadurch gekennzeichnet, dass** die Anpressscheibe (31) über eine Klemmverbindung, z.B. eine Konusklemmung oder Keilklemmung, am Grundteil (13) festklemmbar ist

10. Gelenkprothese nach einem der Ansprüche 1 bis 9, **dadurch gekennzeichnet, dass** die Anpressscheibe (31) ringförmig ist.

11. Gelenkprothese nach einem der Ansprüche 1 bis 9, **dadurch gekennzeichnet, dass** die Anpressscheibe (31) C-förmig ist

12. Gelenkprothese nach einem der Ansprüche 1 bis 11, **dadurch gekennzeichnet, dass** am Anlenkkopf (25) und an der Anpressscheibe (31,53) vorliegende, zusammenwirkende Berührungsflächen (z.B. 37,69,73,49,99) bezüglich Form und Material derart ausgebildet sind, dass sich ein oder mehrere Berührungsbereiche zwischen diesen Berührungsflächen (z.B. 37,69,73,49,99) ergeben, in denen unter Einwirkung der beim Anpressen auftretenden Druckkräfte wenigstens eine der Berührungsflächen (z.B. 37,69,73,49,99) plastisch verformbar ist.

13. Gelenkprothese nach Anspruch 12, **dadurch gekennzeichnet, dass** wenigstens eine punktuelle Berührung oder eine durchgehende oder unterbrochene Linienberührung zwischen Anlenkkopf (25) und Anpressplatte (31,53) besteht

14. Gelenkprothese nach Anspruch 12 oder 13, **dadurch gekennzeichnet, dass** die Berührungsbereiche auf einer Kugeloberfläche liegen.

15. Gelenkprothese nach einem der Ansprüche 1 bis 11, **dadurch gekennzeichnet, dass** am Anlenkkopf (25) und in einem Anlenkraum (19) vorliegende Berührungsflächen (z.B. 23,23',49,50,57,67,71,75,81,99) derart ausgebildet sind, dass wenigstens eine auf einer Kugeloberfläche liegende punktuelle Berührung oder eine durchgehende oder unterbrochene Linienberührung zwischen einer Klemmfläche (61,71) des Anlenkraums (19) und dem Anlenkkopf (25) besteht

16. Gelenkprothese nach einem der Ansprüche 12 bis 15, **dadurch gekennzeichnet, dass** eine der Berührungsflächen (z.B. 23,23',37,49,57,69,71,73, 81,99) durch eine oder mehrere Körperkanten oder eine oder mehrere Körperspitzen, und die andere Berührungsfläche (50,67,69,75) durch eine glatte oder rauhe Kugeloberfläche gebildet ist

17. Gelenkprothese nach Anspruch 15 und 16, **dadurch gekennzeichnet, dass** eine mit der Kugeloberfläche zusammenwirkende Berührungsfläche Kanten (49, 75, 81, 99) bildet, welche quer zu einer Rotationsrichtung des Halsteils (21) um eine Halsachse (33) liegen.

18. Gelenkprothese nach einem der Ansprüche 1 bis 17, **dadurch gekennzeichnet, dass** ein Anlenkraum (19) in einem Deckel oder Überwurfmutter (53) ausgebildet ist, der vorzugsweise mit einem Innengewinde auf ein Aussengewinde am Schaftteil (13) schraubbar ist

## Claims

1. A joint prosthesis (11) with an artificial joint socket (12) for anchoring in a first bone or for pivotal co-operation with a natural joint socket in the first bone, an axial base piece (13) for anchoring in a second bone, an articulation head (25) articulated thereon and forming, together with a collar extension (27) disposed thereon and defining a collar axis (33), a collar piece (21) which is pivotable and rotatable relatively to the base piece (13) about at least two axes passing through the articulation head (25) and perpendicular to one another, a head cap (29) disposed on the collar extension (27) and co-operating as a joint with the joint socket (12), at least one pressure piece (31, 53) for pressing the articulation head (25) against the base piece (13), and means (e.g. 43, 53, 39, 41, 51, 107) for connecting the pressure piece (31, 53) and the base piece (13) so that the collar extension (25) is lockable in any desired selected rotary position and angular position to the axis of the base piece (13), between the pressure piece (31, 53) and the base piece (13), **characterized in that** the pressure piece (31, 53) comprises a pressure disc (31, 53) with a passage opening (57) for the collar extension (27).

2. A joint prosthesis according to claim 1, **characterized in that** the pressure disc (31) is rotatable relatively to the base piece (13) about an axis through the passage opening (35).

3. A joint prosthesis according to claim 1, **characterized in that** the pressure disc (31, 53) is non-rotatable relatively to the base piece (13) with respect to an axis through the passage opening (35).

4. A joint prosthesis according.to any one of claims 1 to 3, **characterized in that** the pressure disc (31) is fixed on the base piece (13) so as to be pivotable about an axis transversely of the collar axis (33).

5. A joint prosthesis according to any one of claims 1 to 4, **characterized in that** the pressure disc (31, 53) and base piece (13) are adapted to be screwed together.

6. A joint prosthesis according to claim 5, **characterized in that** the pressure disc is a cap nut (53) or is fixable with a cap nut (53).

7. A joint prosthesis according to claim 6, **characterized in that** the cap nut (53) is connectable to the base piece (13) by a bayonet fastener.

8. A joint prosthesis according to any one of claims 1 to 5, **characterized in that** the pressure disc (31) is connectable to the base piece (13) by resilient clamping means (107).

9. A joint prosthesis according to any one of claims 1 to 4, optionally in conjunction with claim 8, **characterized in that** the pressure disc (31) is adapted to be clamped on the base piece (13) via a clamping connection, for example a cone or wedge clamping.

10. A joint prosthesis according to any one of claims 1 to 9, **characterized in that** the pressure disc (31) is annular.

11. A joint prosthesis according to any one of claims 1 to 9, **characterized in that** the pressure disc (3 1) is C-shaped.

12. A joint prosthesis according to any one of claims 1 to 11, **characterized in that** co-operating contact surfaces (e.g. 37, 69, 73, 49, 99) present on the articulation head (25) and on the pressure disc (31, 53) are so constructed in respect of shape and material that one or more contact zones form between said contact surfaces (e.g. 37, 69, 73, 49, 99), in which zones at least one of the contact surfaces (e.g. 37, 69, 73, 49, 99) is plastically deformable under the action of the pressure forces occurring during pressing.

13. A joint prosthesis according to claim 12, **characterized in that** at least one punctiform contact or one continuous or interrupted linear contact exists between the articulation head (25) and the pressure plate (31, 33).

14. A joint prosthesis according to claim 12 or 13, **characterized in that** the contact zones lie on a spherical surface.

15. A joint prosthesis according to any one of claims 1 to 11, **characterized in that** contact surfaces (e.g. 23, 23', 49, 50, 57, 67, 71, 75, 81, 99) present on the articulation head (25) and in an articulation chamber (19) are so constructed that at least one point contact lying on a spherical surface or a continuous or interrupted linear contact exists between a clamping surface (61, 71) of the articulation chamber (19) and the articulation head (25).

16. A joint prosthesis according to any one of claims 12 to 15, **characterized in that** one of the contact surfaces (e.g. 23, 23', 37, 49, 57, 69, 71, 73, 81, 99) is formed by one or more body edges or by one or more body points and the other contact surface (50, 67, 69, 75) is formed by a smooth or rough spherical surface.

17. A joint prosthesis according to claim 15 and 16, **characterized in that** a contact surface co-operating with the spherical surface forms edges (49, 75, 81, 99) which lie transversely of a rotational direction of the collar piece (21) about a collar axis (33).

18. A joint prosthesis according to any one of claims 1 to 17, **characterized in that** an articulation chamber (19) is formed in a cover or cap nut (53) which is preferably screwable by an internal screwthread on to an external screwthread on the shaft piece (13).

## Revendications

1. Prothèse d'articulation (11) avec une cavité articulaire artificielle (12) pouvant être ancrée dans un premier os ou pour l'action conjointe articulée avec une cavité articulaire naturelle dans le premier os, une pièce de base axiale (13) pouvant être ancrée dans un second os, une tête d'articulation (25) articulée sur celle-ci qui forme, avec un prolongement de col (27) placé sur celle-ci définissant un axe de col (33), une partie de col (21) qui est rotative et pivotante par rapport à la pièce de base (13) autour d'au moins deux axes perpendiculaires l'un à l'autre, traversant la tête d'articulation (25), une calotte de tête (29) qui est placée sur le prolongement de col (27), agissant conjointement de manière articulée avec la cavité articulaire (12), au moins une pièce de compression (31, 53) pour presser la tête d'articulation (25) contre la pièce de base (13) et des moyens (par exemple 43, 53, 39, 41, 51, 107) pour relier la pièce de compression (31, 53) et la pièce de base (13) si bien que le prolongement de col (25) peut être bloqué dans n'importe quelle position de rotation et position angulaire pouvant être choisie par rapport à l'axe de la pièce de base (13) entre la pièce de compression (31, 53) et la pièce de base (13),
**caractérisée en ce**
**que** la pièce de compression (31, 53) présente un disque de compression (31, 53) avec une ouverture de passage (37) pour le prolongement de col (27).

2. Prothèse d'articulation selon la revendication 1, **caractérisée en ce que** le disque de compression (31) est rotatif par rapport à la pièce de base (13) autour d'un axe à travers l'ouverture de passage (35).

3. Prothèse d'articulation selon la revendication 1, **caractérisée en ce que** le disque de compression (31, 53) n'est pas rotatif par rapport à la pièce de base (13) par rapport à un axe à travers l'ouverture de passage (35).

4. Prothèse d'articulation selon l'une des revendications 1 à 3,
**caractérisée en ce que** le disque de compression (31) est fixé sur la pièce de base (13) en étant pivotant autour d'un axe transversalement par rapport à l'axe du col (33).

5. Prothèse d'articulation selon l'une des revendications 1 à 4,
**caractérisée en ce que** le disque de compression (31, 53) et la pièce de base (13) peuvent être vissés l'un à l'autre.

6. Prothèse d'articulation selon la revendication 5, **caractérisée en ce que** le disque de compression est un écrou-raccord (53) ou peut être fixé à un écrou-raccord (53).

7. Prothèse d'articulation selon la revendication 6, **caractérisée en ce que** l'écrou-raccord (53) peut être relié à la pièce de base (13) avec une fermeture à baïonnette.

8. Prothèse d'articulation selon l'une des revendications 1 à 5,
**caractérisée en ce que** le disque de compression (31) peut être relié à la pièce de base (13) par des moyens de serrage élastiques (107).

9. Prothèse d'articulation selon l'une des revendications 1 à 4, tout au plus en relation avec la revendication 8, **caractérisée en ce que** le disque de compression (31) peut être bloqué sur la pièce de base (13) par une jonction par serrage, par exemple par un serrage de cône ou de clavette.

10. Prothèse d'articulation selon l'une des revendications 1 à 9,
**caractérisée en ce que** le disque de compression (31) est annulaire.

11. Prothèse d'articulation selon l'une des revendications 1 à 9,
**caractérisée en ce que** le disque de compression (31) est en forme de C.

12. Prothèse d'articulation selon l'une des revendications 1 à 11,
**caractérisée en ce que** des surfaces de contact (par exemple 37, 69, 73, 39, 99) à action conjointe existant sur la tête d'articulation (25) et sur le disque de compression (31, 53) sont configurées pour ce qui est de la forme et du matériau de telle manière qu'il résulte une ou plusieurs zones de contact entre ces surfaces de contact (par exemple 37, 69, 73, 39, 99) dans lesquelles, sous l'effet des forces de pression qui interviennent lors de la pression, au moins l'une des surfaces de contact (par exemple 37, 69, 73, 39, 99) peut être déformée plastiquement.

13. Prothèse d'articulation selon la revendication 12, **caractérisée en ce qu'**il y a au moins un contact ponctuel ou un contact en ligne continue ou interrompue entre la tête d'articulation (25) et la plaque de compression (31, 53).

14. Prothèse d'articulation selon la revendication 12 ou 13,
**caractérisée en ce que** les zones de contact se trouvent sur une surface sphérique.

15. Prothèse d'articulation selon l'une des revendications 1 à 11,
**caractérisée en ce que** des surfaces de contact (par exemple 23, 23', 49, 50, 57, 67, 71, 75, 81, 99) existant sur la tête d'articulation (25) et dans un espace d'articulation (19) sont configurées de telle manière qu'il y a au moins un contact ponctuel situé sur une surface sphérique ou un contact en ligne continue ou interrompue entre une surface de serrage (61, 71) de l'espace d'articulation (19) et la tête d'articulation (25).

16. Prothèse d'articulation selon l'une des revendications 12 à 15,
**caractérisée en ce qu'**une des surfaces de contact (par exemple 23, 23', 49, 50, 57, 67, 71, 75, 81, 99) est formée par une ou plusieurs arêtes de corps ou une ou plusieurs pointes de corps et l'autre surface de contact (50, 67, 69, 75) est formée par une surface sphérique lisse ou rugueuse.

17. Prothèse d'articulation selon la revendication 15 ou 16,
**caractérisée en ce qu'**une surface de contact à action conjointe avec la surface sphérique forme des arêtes (49, 75, 81, 99) qui se trouvent transversalement par rapport à un sens de rotation de la partie de col (21) autour d'un axe de col (33).

18. Prothèse d'articulation selon l'une des revendications 1 à 17,
**caractérisée en ce qu'**un espace d'articulation (19) est configuré dans un couvercle ou un écrou-raccord (53) qui peut être vissé de préférence avec un filet intérieur sur un filet extérieur sur la partie tige (13).
